# DEMANDE DE BREVET EUROPEEN

(11) **EP 0 638 647 A1**
(43) Date de publication de la demande: **15.02.1995**
(21) Numéro de dépôt: 94401715.1
(22) Date de dépôt: 26.07.1994
(51) Int. Cl.: C12N 15/86, C12N 15/85

(54) **Sequences nucléotidiques permettant la replication et le maintien d'une information génétique dans les cellules animales**

(30) Priorité: 26.07.1993 FR 9309156
(71) Demandeur: INSTITUT NATIONAL DE LA RECHERCHE AGRONOMIQUE (INRA), F-75341 Paris Cédéx 07 (FR); CENTRE NATIONAL DE LA RECHERCHE SCIENTIFIQUE (CNRS), 75794 Paris Cédex 16 (FR)
(72) Inventeur: Devauchelle, Gérard, F-30380 Saint Christol-les-Ales (FR); Cerutti, Martine, F-30380 Saint Christol-les-Ales (FR); Persillon, Cécile, F-30100 Ales (FR)
(74) Mandataire: Vialle-Presles, Marie José

(57) **Abrégé**

L'Invention a pour objet l'utilisation d'un fragment d'ADN comprenant une séquence de l'extrémité 3' du gène *IE1* du baculovirus AcNPV, optionnellement associé à un fragment d'ADN comprenant une séquence située en amont du promoteur dudit gène *IE1*, pour l'obtention de vecteurs permettant la réplication et le maintien d'une information génétique dans les cellules animales.

L'Invention englobe également les vecteurs épisomaux obtenus, ainsi que les cellules transformées comprenant lesdits vecteurs.

## Description

La présente Invention est relative à des vecteurs permettant la réplication et le maintien d'une information génétique dans des cellules animales, en particulier les cellules d'insectes.

L'utilisation de cellules eucaryotes, et en particulier de cellules animales, pour l'expression de gènes hétérologues clonés fait l'objet de nombreuses recherches. En effet, seules les cellules eucaryotes sont capables de procéder aux modifications post-traductionnelles qui sont dans de nombreux cas nécessaires pour obtenir une protéine active. Actuellement, la levure constitue un des systèmes d'expression eucaryote les plus utilisés, pour sa facilité de mise en oeuvre ; toutefois, le système de glycosylation des levures, qui diffère quelque peu de celui des cellules animales ne permet pas l'obtention de certaines protéines sous forme active.

Pour obtenir l'expression d'un gène dans une cellule animale, on utilise en particulier des vecteurs d'origine virale. Parmi ceux-ci on citera les baculovirus qui sont actuellement employés dans de très nombreux laboratoires. En effet, ces virus possèdent les avantages suivants ; ils permettent l'insertion de longs segments d'ADN et possèdent en outre plusieurs promoteurs forts, actifs à différents stades du cycle de réplication du virus. Ces promoteurs sont capables d'induire un niveau d'expression extrêmement élevé des gènes placés sous leur contrôle. Deux promoteurs tardifs, le promoteur de la polyédrine, et le promoteur de la protéine P10, ont été plus particulièrement utilisés pour l'expression de gènes hétérologues.

Cependant, ces vecteurs d'expression dérivés de baculovirus possèdent certains inconvénients qui limitent leur usage : tout d'abord, le gène hétérologue inséré dans l'un de ces vecteurs ne s'exprime que dans le cadre d'une infection virale ; or, l'infection virale aboutit rapidement à la lyse des cellules, ce qui implique que l'expression d'un gène dans celles-ci ne peut être que transitoire, et ce qui entraîne par ailleurs la dégradation rapide des protéines produites par la cellule. En outre, dans la mesure où le gène hétérologue est placé sous contrôle d'un promoteur tardif, il ne s'exprime qu'en fin de cycle de réplication du virus, à un stade où les fonctions cellulaires sont déjà altérées, et en particulier les fonctions assurant les modifications post-traductionnelles (par exemple la glycosylation).

Certaines équipes ont cherché à résoudre ces problèmes en insérant le gène hétérologue dont l'expression est recherchée, sous contrôle non plus d'un promoteur tardif, mais d'un promoteur précoce. Les gènes placés sous contrôle de promoteurs précoces s'expriment fortement dès le début de l'infection virale. Certains de ces promoteurs, par exemple les promoteurs des gènes *IE1* [GUARINO et SUMMERS, J. Virol. 61 : 7, p. 2091-2099 (1987)], *IE0*, et *IEN* du baculovirus AcNPV (virus de la polyhédrose nucléaire d'*Autographa californica*) sont, contrairement aux promoteurs de la polyédrine ou de P10, parfaitement reconnus par l'ARN polymérase II de la cellule. L'expression de gènes placés sous le contrôle de ces promoteurs ne dépend donc pas de l'infection virale. I1 a ainsi été proposé d'utiliser les promoteurs *IE1* et *IEN* pour exprimer très précocément des gènes hétérologues.

Il a aussi été proposé d'utiliser la séquence codant pour la protéine IE1, en association avec un promoteur d'un autre gène précoce de baculovirus, tel que le promoteur 39K (la protéine codée par le gène *IE1* est un facteur de trans-activation de l'expression d'autres gènes précoces de baculovirus, en particulier du gène 39K), et éventuellement avec des séquences amplificatrices, (hr1, hr2, hr3, hr4, hr5) [GUARINO et al., J. Virol., 60:1, p. 224-229, 1986)] pour la construction de vecteurs d'expression ; (Demande PCT/WO 92/05265 au nom de THE TEXAS A & M UNIVERSITY SYSTEM désignant comme Inventeurs GUARINO et JARVIS).

Le but poursuivi, dans les deux cas, était d'obtenir en particulier des vecteurs pouvant s'intégrer dans le génome cellulaire pour exprimer de façon stable un gène hétérologue sous contrôle d'un promoteur précoce de baculovirus. Cependant, l'utilisation de tels vecteurs pose d'autres problèmes. En effet, ni le site ni la stabilité de l'intégration dans le génome cellulaire ne sont contrôlables ; or, ces deux paramètres conditionnent dans une large mesure l'expression du gène intégré.

Une autre approche des problèmes posés par l'expression d'une protéine hétérologue dans une cellule animale, consiste à tenter de construire *de novo* un vecteur d'expression qui d'une part pourrait se répliquer de manière autonome, et d'autre part pourrait se maintenir dans la cellule et se transmettre à sa descendance. Ceci implique que ce vecteur comporte au moins une séquence assurant la fonction d'une origine de réplication active dans la cellule, ainsi que des séquences assurant son maintien dans la cellule, et sa ségrégation dans les cellules-filles au cours de la division cellulaire.

On connait depuis longtemps, chez la levure, des séquences actives comme origine de réplication extrachromosomique : ces séquences présentés sur des portions d'ADN sont dénommées ARS (autonomously replicating sequences). Les éléments ARS possèdent deux séquences consensus : une séquence coeur (domaine A) qui est nécessaire pour la fonction ARS [KEARSEY, Cell., 37, 299-307, (1984)] mais ne peut par elle-même suffire à permettre la réplication autonome, et nécessite la présence de séquences flanquantes additionnelles, et une séquence consensus 3' (domaine C) située en aval de l'extrémité 3' du brin riche en résidus T de la séquence coeur.

Bien que des séquences similaires aux séquences consensus des éléments ARS aient été décrites dans des cellules animales, leur rôle éventuel dans la réplication de l'ADN dans ces cellules n'a jamais été établi. Plusieurs études ont été effectuées dans le but d'identifier des séquences effectivement actives comme origine de réplication dans des cellules animales, en particulier dans des cellules de mammifères [KRYSAN et al., Mol. Cell. Biol. 9, p. 1026-1033, (1989) ; HOLST et al., Cell 52, p. 355-365, (1988) ; cf aussi revue par UMEK et al., Biochim. and Biophys. Acta, 1007, p. 1-14, (1989)]. Toutefois dans la plupart des cas, ces études n'ont pas abouti à l'obtention de séquences permettant la réplication et le maintien d'information génétique extrachromosomique.

Dans le cas des cellules d'insecte, la seule origine de réplication qui a été identifiée est celle de l'ADN mitochondrial de *Drosophila melanogaster* [SUGINO, Biochem. Biophys. Res. Commun. 91, p. 1321-1329, (1979)]. Bien que des séquences ARS aient également été localisées dans le génome de la drosophile [GRAGEROV et al., Nucl. Acids Res. 16, p. 1169-1180, (1988)], leur éventuelle activité en tant qu'origine de réplication n'a pas été montrée. En ce qui concerne les virus d'insectes, la présence, en au moins 2 régions du génome du baculovirus AcNMPV, de séquences qui se comportent comme les éléments ARS quand elles sont introduites dans des cellules de levure, a été décrite [HOOFT VAN HIDDEKINGE et al., Arch. Virol. 88, p. 279-284, (1986)]. Plus récemment, 4 fragments contenant des ARS fonctionnelles chez *S. cerevisiae* ont été identifiés [LEE et al., Virus Research, 24, p. 249-264, (1992)] dans le génome d'un autre baculovirus : le baculovirus NPV de *Christoneura fumiferana*. L'analyse de la séquence de celui de ces fragments qui possède l'activité ARS la plus forte, montre une région riche en A+T associée avec une région d'ADN courbé. Bien qu'aucune séquence ne corresponde exactement à la séquence consensus coeur des éléments ARS de levure, 13 régions de séquences proches de celle de ces éléments (9 ou 10 paires de bases sur 11) ont été identifiées. Cependant, aucune indication n'est donnée sur la fonctionnalité de ces séquences dans des cellules eucaryotes autres que des cellules de levure. En particulier, elles ne sont pas supposées intervenir dans la réplication virale, d'autant plus qu'il a été montré récemment [PEARSON et al., Science, 257, p. 1382-1384, (1992)] que chez le baculovirus AcNPV, des origines de réplication de l'ADN sont situées au niveau des séquences répétées, dénommées séquences hr, dont chacune comprend plusieurs palindromes imparfaits très semblables entre eux. Ces séquences, qui sont présentes dans 6 régions distribuées le long du génome de AcNPV, étaient connues auparavant pour leurs propriétés d'amplificateurs d'expression [cf. publication de GUARINO et al. (1986) précitée]. L'analyse par délétion des séquences hr a permis de démontrer qu'une séquence contenant un seul palindrome complet permettait la réplication. D'autres auteurs [KOOL et al., Virology, 192, p. 94-101, (1993)] ont décrit la détection et l'analyse fonctionnelle de séquences présentes dans l'ADN de virus défectifs, et qui peuvent servir d'origine de réplication pour AcNPV dans les cellules infectées. L'activité liée à la réplication virale pourrait être localisée essentiellement dans la région de 1000 paires de bases contenant l'ADN hautement répétitif hr5.

Dans le but de construire des vecteurs stables capables de se répliquer de manière autonome dans des cellules de lépidoptères, les Inventeurs ont dans un premier temps entrepris la recherche d'une origine de réplication active dans lesdites cellules.

Pour cela, ils ont construit un plasmide (dénommé ci-après pIE1NéoR) contenant un gène rapporteur de résistance à la néomycine, inséré sous contrôle du promoteur du gène IE1 du baculovirus AcNPV, dans l'intention de l'utiliser pour la construction et le criblage d'une banque d'ADN génomique de cellules de *Spodoptera frugiperda* (Sf9) afin de rechercher des séquences d'ADN cellulaire portant une origine de réplication active dans des cellules d'insectes.

Or au cours de ce travail, les Inventeurs ont constaté que, de façon surprenante, le plasmide pIE1NéoR était capable, en l'absence de tout insert d'ADN d'origine cellulaire, de se répliquer de manière autonome dans les cellules, et qu'en outre cette réplication s'accompagnait à l'issue de quelques passages en culture, d'une amplification et d'une structuration du matériel génétique plasmidique sous forme d'une structure extra-chromosomique de haut poids moléculaire dans la descendance des cellules initialement transformées.

Les Inventeurs ont recherché quelles étaient les régions du plasmide qui étaient responsables de ces propriétés, et ont découvert qu'elles étaient associées à la présence d'une séquence d'ADN chevauchant la portion du gène IE1 codant pour l'extrémité COOH terminale de la protéine IE1 ainsi que la région 3' flanquante de ce gène. Cette séquence, qui sera également dénommée dans ce qui suit "séquence AMIG" (Amplification et Maintien d'Information Génétique), est indiquée dans la liste des séquences en annexe sous le numéro SEQ.ID N°1.

En procédant à l'analyse de la séquence AMIG, dans le but de localiser les structures responsables de ces propriétés, les Inventeurs ont mis en évidence plusieurs séquences apparentées aux séquences ARS de la levure, dont une séquence MCCS de 11 pb homologue à la séquence consensus du domaine A des éléments ARS. Ils ont également localisé des séquences voisines des séquences centromériques consensus de la levure et des cellules de mammifère.

D'autre part, les Inventeurs ont mis en évidence une séquence qui est située en amont du promoteur du gène IE1, et constaté que cette séquence intervient pour assurer la stabilité de la structure extra-chromosomique de haut poids moléculaire. Cette séquence, qui sera dénommée dans ce qui suit "séquence STAB", est indiquée dans la liste des séquences en annexe sous le numéro SEQ ID NO:2.

Différents plasmides, comprenant les séquences AMIG et STAB, en association avec divers promoteurs ont été construits ; tous ces plasmides possédent les propriétés mentionnées ci-dessus, à savoir qu'ils sont capables de se répliquer dans les cellules transformées, et en outre de se maintenir dans leur descendance, et de se structurer sous forme d'épisomes de très haut poids moléculaire.

Dans l'exposé de la présente Invention, on entend par "épisome" une molécule d'ADN extrachromosomique et auto-réplicative.

La présente Invention a pour objet l'utilisation un fragment d'ADN comprenant la séquence indiquée dans la liste des séquences en annexe sous le numéro SEQ.ID.N°1, pour l'obtention d'un vecteur capable de se répliquer de manière autonome dans une cellule animale et de se maintenir dans la descendance de ladite cellule sous forme d'épisome.

Selon un mode de réalisation préféré de la présente Invention, on utilise le fragment d'ADN de séquence SEQ.ID.N°1 en association avec un fragment d'ADN comprenant la séquence indiquée dans la liste des séquences en annexe sous le numéro SEQ.ID.N°2.

La présente Invention a en outre pour objet un vecteur capable de se multiplier dans une cellule animale et de se maintenir dans la descendance de ladite cellule, caractérisé en ce qu'il est sous forme d'épisome constitué par un concatémère d'un vecteur-unité, lequel vecteur-unité comprend la séquence indiquée dans la liste des séquences en annexe sous le numéro SEQ.ID.N°1.

Selon un mode de réalisation préféré de la présente Invention, ledit vecteur-unité comprend en outre la séquence indiquée dans la liste des séquences en annexe sous le numéro SEQ. ID.N°2.

Au sens de la présente invention on entend par "concatémère" une molécule d'ADN constituée par au moins 2 copies de l'information génétique portée par le vecteur-unité. Le "vecteur-unité" peut comprendre, en particulier, outre la séquence SEQ ID NO:1, et avantageusement, la séquence SEQ ID NO:2, au moins un fragment d'ADN dont on souhaite obtenir la multiplication dans une cellule animale et le maintien dans la descendancé de ladite cellule. Il peut s'agir en particulier de fragments d'ADN portant l'information génétique nécessaire à la production de protéines, ou constituant un marqueur de sélection, etc...
La présente Invention a également pour objet un procédé d'obtention de lignées cellulaires animales capables de transmettre à leur descendance une information génétique extra-chromosomique d'origine hétérologue, lequel procédé est caractérisé en ce qu'il comprend une étape au cours de laquelle l'on procède à la transfection d'une cellule animale par une préparation d'ADN comprenant au moins un vecteur-unité portant ladite information génétique et comprenant la séquence indiquée dans la liste des séquences en annexe sous le numéro SEQ. ID.N°1, ou par une préparation d'ADN comprenant au moins un épisome constitué d'un concatémère dudit vecteur-unité, et une étape au cours de laquelle on procède à la multiplication de ladite cellule animale, et à la sélection des descendants de ladite cellule portant au moins un épisome constitué d'un concatémère dudit vecteur-unité.

Selon un mode de mise en oeuvre préféré du procédé conforme à l'Invention, le vecteur-unité comprend en outre la séquence indiquée dans la liste des séquences en annexe sous le numéro SEQ.ID.N°2.

Il est possible de préparer des vecteurs épisomaux conformes à l'Invention par extraction d'ADN à partir des cellules sélectionnées à l'issue du procédé décrit ci-dessus.

Pour obtenir un vecteur-unité, permettant la mise en oeuvre des procédés d'obtention de lignées cellulaires animales et de vecteurs épisomaux conformes à l'invention, on procède à la ligation, dans un vecteur-hôte approprié, de la séquence indiquée dans la liste des séquences en annexe sous le numéro SEQ.ID.NO:l, et avantageusement, de la séquence indiquée dans la liste des séquences en annexe sous le numéro SEQ.ID.NO:2, ainsi que de toute autre séquence d'ADN dont on souhaite obtenir la multiplication dans une cellule animale et le maintien dans la descendance de ladite cellule. Les méthodes permettant l'obtention de ce vecteur-unité sont des méthodes classiques de génie génétique, utilisant des vecteurs hôtes d'utilisation courante, telles que celles décrites par SAMBROOK et al. [Molecular Cloning : A Laboratory Manual, Seconde édition ; Cold Spring Harbor Laboratory Press (1989)].

L'objet de la présente Invention englobe également des lignées cellulaires stables capables d'exprimer une information génétique d'origine hétérologue, lesquelles lignées sont caractérisées en ce que chaque cellule de ladite lignée contient au moins un vecteur épisomal conforme à l'Invention.

Selon un mode de réalisation préféré de la présente Invention, lesdites lignées cellulaires sont des lignées de cellules d'insectes, en particulier de lépidoptères.

La présente Invention permet d'obtenir un système d'expression qui assure, de façon stable, l'expression d'une information génétique d'origine hétérologue dans des cellules animales. Ce système est utilisable en particulier pour la production de protéines recombinantes, car il permet d'obtenir, en continu et en assurant un niveau d'expression élevé, la protéine d'intérêt, indépendamment de l'intégration du gène codant pour celle-ci dans le génome cellulaire, donc sans les aléas liés à cette intégration.

La présente Invention sera mieux comprise à l'aide du complément de description qui va suivre, qui se réfère à des exemples d'obtention et d'utilisation de vecteurs comprenant les séquences AMIG.

Il va de soi toutefois que ces exemples sont donnés uniquement à titre d'illustration de l'objet de l'Invention, dont ils ne constituent en aucune manière une limitation.

Les techniques générales de biologie moléculaire auxquelles il est fait référence dans les exemples qui suivent sont celles décrites par SAMBROOK et al. [Molecular Cloning : A Laboratory Manual, Seconde édition ; Cold Spring Harbor Laboratory Press (1989)].

### EXEMPLE 1 : CONSTRUCTION D'UN PLASMIDE COMPRENANT LES SEQUENCES AMIG ET STAB ; REPLICATION DUDIT PLASMIDE DANS DES CELLULES D'INSECTES ET FORMATION DE STRUCTURES EPISOMALES DE TRES HAUT POIDS MOLECULAIRE

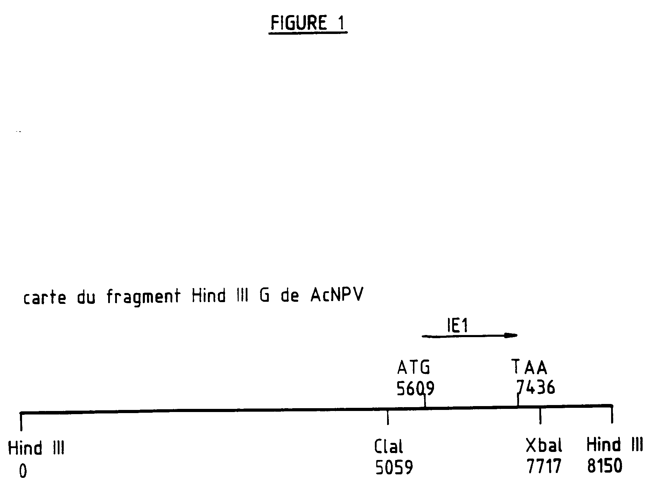

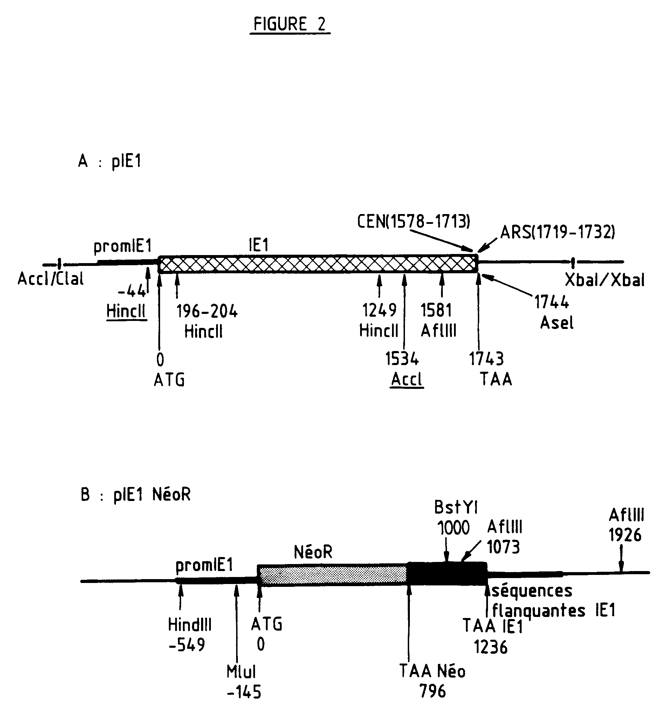

Le gène *IE1* et son promoteur sont situés dans le sous-fragment ClaI-XbaI (2658 pb) du fragment HindIII-G du baculovirus AcNPV (représenté à la Figure 1). Ce sous-fragment ClaI-XbaI a été excisé, puis inséré dans le plasmide pUC18, à la place du fragment AccI-XbaI de celui-ci : on obtient de la sorte le plasmide pIE1 (représenté à la Figure 2A) : (Ces bases sont numérotées à partir du A du codon ATG de IE1).

Le gène de résistance à la néomycine (NéoR) a été inséré dans le plasmide pIE1 en aval du promoteur du gène codant pour la protéine IE1, de la manière suivante : le fragment BglII-SmaI de pNéo (PHARMACIA) a été inséré à la place du fragment HincII-AccI de pIE1. Les extrémités du plasmide pIE1 et l'extrémité Bg1II du fragment portant le gène *NéoR* ont, préalablement à la jonction, été rendues franches par action de la polymérase de Klénow. Le plasmide ainsi obtenu est dénommé pIE1NéoR : l'insert IE1NéoR est schématisé à la Figure 2B : (Ces bases sont numérotées à partir du A de l'ATG de NéoR..

Le plasmide pIE1NéoR a été utilisé pour transformer E. coli DH5α F'IQ (GIBCO-BRL), et purifié sur gradient de CsC1 à partir des bactéries recombinantes sélectionnées sur la base de leur résistance à l'ampicilline.

Le plasmide pIE1NéoR a été introduit par lipofection dans les cellules de *Spodoptera frugiperda* (clone Sf9) selon le protocole suivant :

Deux millions de cellules sont placées dans une fiole de 25 cm² dans du milieu TC100 (GIBCO) supplémenté par 10% (v/v) de sérum de veau foetal. Au bout d'une heure à température ambiante, les cellules se sont collées au fond de la fiole. On retire le milieu et on le remplace par 3 ml du même milieu additionné de 40 µl de DOTAP (BOERHINGER) et de 10 µg de plasmide pIE1NéoR. Après quatre heures d'incubation à 28°C, le milieu est de nouveau remplacé par 4 ml du même milieu (TC100 + 10%SVF), et comprenant 1 mg/ml de G418 (GIBCO-BRL). Après plusieurs passages en culture dans ce milieu, les cellules sont clonées sur des boîtes de Pétri de 140 mm de diamètre (environ 100 cellules/boîte), dans le même milieu contenant 3% de méthyl cellulose 3000-5000 (PROLABO) et 1 mg/ml de G418. Les colonies résistantes au G418 sont sélectionnées.

Dans le but de caractériser la structure portant l'information génétique de pIE1NéoR, l' ADN total des cellules résistantes au G418 a été analysé par électrophorèse en champ pulsé selon le protocole suivant : 2 millions de cellules sont récoltées, lavées 2 fois avec du PBS et incluses dans de l'agarose à 1%. Après polymérisation à 4°C, les blocs d'agarose sont immergés dans 5 volumes de tampon de lyse (10 mM de Tris-HCl pH 8, 100 mM EDTA, 1% N-lauroylsarcosine, 200 µg/ml protéinase K) pendant 16 heures à 50°C. Les blocs sont ensuite rincés deux fois dans du tampon TE (10 mM Tris-HCl pH 7,5 ; 1 mM EDTA) puis incubés 2 fois 2 heures à 37°C dans une solution de PMSF (0,5 mM PMSF dans du TE) et rincés à nouveau 3 fois dans du TE. Les blocs sont ensuite équilibrés pendant 16h à 4°C, dans le tampon de l'enzyme de restriction choisie, puis digérés avec 50 U de cette enzyme pendant une nuit. Les blocs ainsi préparés sont directement chargés sur un gel d'agarose à 1% (SEAKEM GTG), et l'on procède à l'électrophorèse, en tampon de migration 0,5 x TBE (45 mM Tris, 32 mM acide borique, 1,25 mM EDTA ; pH 8,3).

La même analyse a été faite sur des cellules témoin Sf9 non transformées.

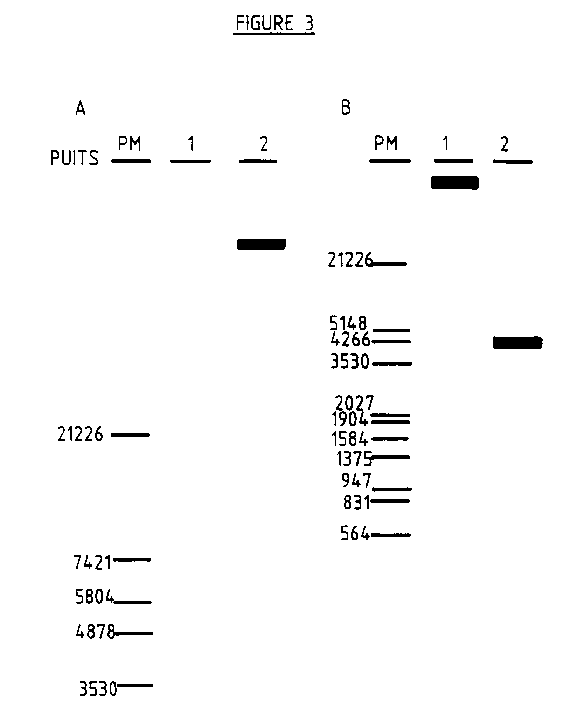

La Figure 3 a été établie d'après les transferts de Southern réalisés à partir des gels d'électrophorèse en champ pulsé de l'ADN de Sf9, après hybridation avec une sonde ³²P ("sonde pIE1NéoR") obtenue par amorçage aléatoire sur pIE1NéoR (Kit de marquage par amorçage aléatoire BOEHRINGER MANNHEIM).
- Figure 3A : Digestion par BglII, migration 18 heures, impulsion 5 secondes, 200 V, gel d'agarose 1% dans du 0,5 x TBE
   . PM : marqueurs de poids moléculaire (exprimé en pb) ;
   . Piste 1 : cellules Sf9 non transfectées (témoin) ;
   . Piste 2 : cellules Sf9 transfectées avec le plasmide pIE1NéoR ;
- Figure 3B : Migration 4 h, impulsion 5 s, 200 V, gel d'agarose 1% dans du TBE
   . PM : Marqueurs de poids moléculaire ;
   . Piste 1 : cellules Sf9 transfectées avec le plasmide pIE1NéoR, ADN non digéré
   . Piste 2 : cellules Sf9 transfectées avec le plasmide pIE1NéoR, ADN digéré par NcoI.

Une forme de très haut poids moléculaire (environ 300 kb) est détectée dans l'ADN non digéré, ou dans l'ADN digéré à l'aide d'une endonucléase qui ne possède pas de site de coupure dans le plasmide pIElnéoR, telle que BglII. Le poids moléculaire apparent est plus élevé dans le cas de l'ADN non digéré que dans celui de l'ADN digéré avec BglII ; ceci s'explique par le fait que dans le cas de l'ADN non digéré, la migration de la structure épisomale est entravée par l'ADN chromosomique de très haut poids moléculaire.

Quand la préparation d'ADN est digérée avec une endonucléase de restriction qui ne possède qu'un seul site de coupure dans la séquence plasmidique de pIE1NéoR, telle que NcoI, un seul fragment correspondant à la taille d'origine du plasmide pIE1néoR est observé.

La bande montrant un signal d'hybridation avec la sonde pEI1néo (piste 2 du gel B) a été excisée, et l'ADN a été extrait et préparé pour l'observation en microscopie électronique. De nombreuses molécules d'ADN circulaires ont été observées, et leur longueur a été estimée à environ 112 µm, ce qui correspond à approximativement 330 kbp.

Ces résultats indiquent la présence, dans les cellules transformées par pIE1NéoR, d'une large structure d'ADN sous forme épisomale, constituée de concatémères de pIE1NéoR associés en tandem direct.

En outre, cette structure peut être extraite des cellules avec une grande efficacité par le procédé de lyse alcaline [CARROL et al. Mol. Cell. BIol., 7, 1740-1750, (1987)], qui est spécifique des structures circulaires quelle que soit leur taille. Cette extraction est effectuée selon le protocole suivant : 5 millions de cellules sont reprises dans 500 µl de tampon de lyse (50 mM NaCl, 2mM EDTA, 1% SDS, pH 12,45), vortexées pendant 2 mn, incubées 30 mn à 30°C. Puis on rajoute 100 µl de Tris-HCl 1M pH 7, 55 µl de NaCl 5M et 5 µl de protéinase K à 10 mg/ml. Ce mélange est incubé 30 mn à 37°C. Après refroidissement, on procède à une extraction au phénol chloroforme et l'ADN est précipité à l'éthanol.

La transfection des cellules Sf9 avec 10 µg de la forme épisomale de pEI1néo (purifiée par la méthode de lyse alcaline), permet d'obtenir des cellules résistantes au G418.

Cette structure épisomale de très haut poids moléculaire apparaît dans les cellules par multimérisation de pIE1NéoR au cours du temps. 25 jours après transfection, pIE1NéoR est détecté dans l'ADN de bas poids moléculaire préparé à partir des cellules résistantes au G418, suivant le protocole de HIRT [J. Mol. Biol. 26, 365-369, (1967)] (qui permet d'obtenir des préparations d'ADN enrichies en structures de faible poids moléculaire), puis séparé sur gel d'agarose à 1%, et apparaît en microscopie électronique sous forme de molécules d'ADN circulaire relachées ou superenroulées, d'une longueur moyenne de 1,6 µm. Cette taille correspond à la taille théorique du plasmide pIE1néoR. D'autre part, l'on a constaté que au fur et à mesure des passages en culture le signal correspondant au plasmide pIE1NéoR (hybridation avec la sonde pIE1NéoR³²P) diminue dans l'ADN extrait par la méthode de HIRT, alors qu'il augmente dans l'ADN cellulaire total. Parallèlement il a été constaté que le nombre de clones obtenus en transformant *E. coli* avec l'ADN extrait des cellules par la méthode de HIRT, diminue significativement avec le temps écoulé depuis la transfection : 200, 2 et 0 clones ont été obtenus avec l'ADN extrait respectivement 25 jours, 40 jours, et 70 jours après la transfection.

### EXEMPLE 2 : CARACTERISATION DES SEQUENCES D'ADN RESPONSABLES DES PROPRIETES DE pIE1NéoR : LOCALISATION DES SEQUENCES AMIG ET STAB

Une série de plasmides ont été obtenus par délétion de fragments de pIE1NéoR :
- pIE1NéoRΔA est obtenu par délétion du fragment compris entre le site AflIII situé à la fin de la séquence codante en 3'de *IE1* et le site AflIII du vecteur pUC18 ;
- pIE1NéoRΔN, pIE1NéoRΔB, et pIE1NéoRΔM résultent de délétions dans la région 5' flanquant le promoteur IE1 :
   * pIE1NéoRΔN est obtenu par délétion du fragment compris entre le site Nhe de *IE1* et le site HindIII du lieur multisite de pUC18 ;
   * pIE1NéoRΔB est obtenu par délétion du fragment compris entre le site BsmI de *IE1* et le site PstI du lieur multisite de pUC18 ;
   * pIE1NéoRΔM est obtenu par délétion du fragment compris entre le site MluI de *IE1* et le site HindIII du lieur multisite de pUC18 ;

Après qu'il ait été vérifié qu'aucune de ces délétions n'affectait la fonction du promoteur *IE1*, les plasmides délétés ont été utilisés pour transfecter les cellules Sf9 comme décrit à l'exemple 1.

Tous les plasmides ont permis l'obtention d'un grand nombre de clones résistants à l'exception du plasmide pIE1NéoRΔA qui n'a permis d'obtenir qu'un petit nombre de clones résistants au G418.

Jusqu'à 100 jours après la transfection, tous les plasmides sont encore été détectés dans l'ADN de bas poids moléculaire préparé à partir des cellules résistantes au G418 suivant le protocole de HIRT ; toutefois, le plasmide pIE1NéoRΔA est détecté en quantité beaucoup plus faible que les autres plasmides.

Dans les expériences réalisées plus tard (300 jours après la transfection), les plasmides ne sont plus détectés dans l'ADN extrait par la méthode de HIRT.

L'ADN des cellules Sf9 résistantes au G418, obtenu par extraction par lyse alcaline, comprend, pour tous les plasmides testés, à l'exception du plasmide pIE1NéoRΔA, des structures circulaires extra-chromosomiques de haut poids moléculaire.

La résistance à la néomycine observée dans les cellules transfectées avec le plasmide pIE1NéoRΔA semble donc résulter de l'intégration aléatoire du plasmide pEI1néoΔA dans l'ADN cellulaire.

Quand l'ADN total préparé à partir de cellules transfectées est digéré avec Bg1II, on observe, avec les plasmides pIE1NéoRΔB, pIE1NéoRΔN ou pIE1NéoRΔM, outre la bande majeure correspondant à la structure épisomale de haut poids moléculaire, de nombreuses bandes additionnelles de plus faible intensité et de plus grande mobilité électrophorétique. Cette observation suggère la présence de concatémères du plasmide intégrés au chromosome (ces bandes additionnelles ne sont pas détectées dans les extraits obtenus par lyse alcaline, ce qui permet de supposer qu'il s'agit de formes intégrées).

D'autre part, des cellules tranfectées avec les différents plasmides ont été sous-clonées et les clones obtenus ont été analysés séparément afin de voir si les résultats observés reflétaient un comportement cellulaire homogène, ou la somme des comportements de sous-populations cellulaires.

Des clones obtenus à partir de cellules transformées par pIE1néoR montrent le même comportement que celui de la population cellulaire polyclonale globale.

Aucun fragment marqué n'a été détecté dans les clones de cellules transformées par pEI1néoΔA, ce qui suggère que si l'information génétique portée par le plasmide est présente, elle ne l'est qu'à un nombre de copies très faible.

Les 3 clones dérivés de pEI1néoΔN ont un comportement identique à celui de la population polyclonale, ce qui suggère la présence, dans une même cellule, de structure épisomales coexistant avec des copies du plasmide qui sont intégrées.

En revanche, parmi les 4 clones dérivés de pIE1NéoRΔB, 2 présentent un comportement similaire aux clones dérivés de pIE1NéoRΔN et 2 présentent un comportement similaire aux clones dérivés de pIE1NéoRΔA. Il en va de même pour les clones obtenus à partir de cellules transformées par le plasmide pIE1NéoRΔM.

Les expérimentations ci-dessus ont permis de localiser les portions du plasmide pIE1NéoR responsables de ses propriétés : le fragment AccI-XbaI de pIE1NéoR, qui comprend la séquence identifiée dans la liste des séquences en annexe sous le numéro SEQ.ID.NO:l (séquence AMIG) porte l'information indispensable pour la réplication plasmidique ; le fragment ClaI-MluI de pIE1NéoR, qui comprend la séquence identifiée dans la liste des séquences en annexe sous le numéro SEQ.ID.NO:2 (séquence STAB) porte une information qui intervient pour favoriser la structuration du plasmide sous forme d'épisome, et pour stabiliser la structure épisomale.

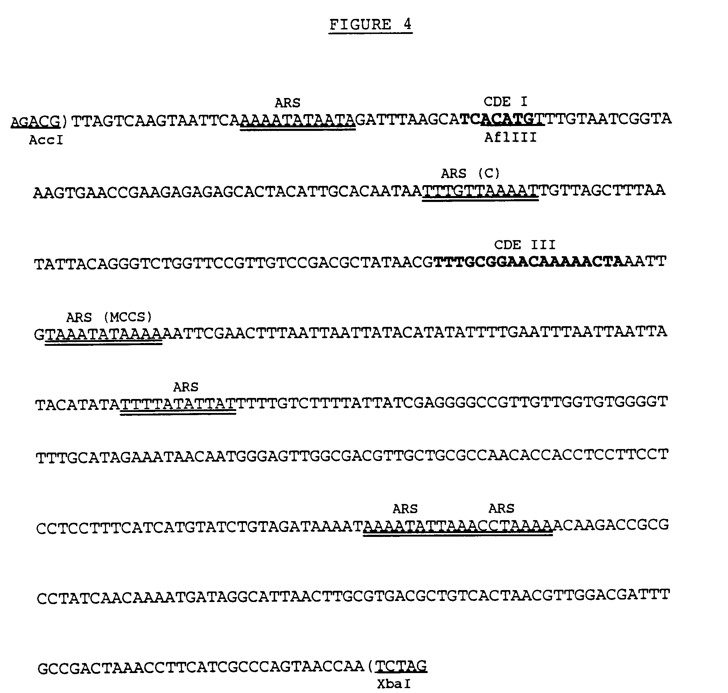

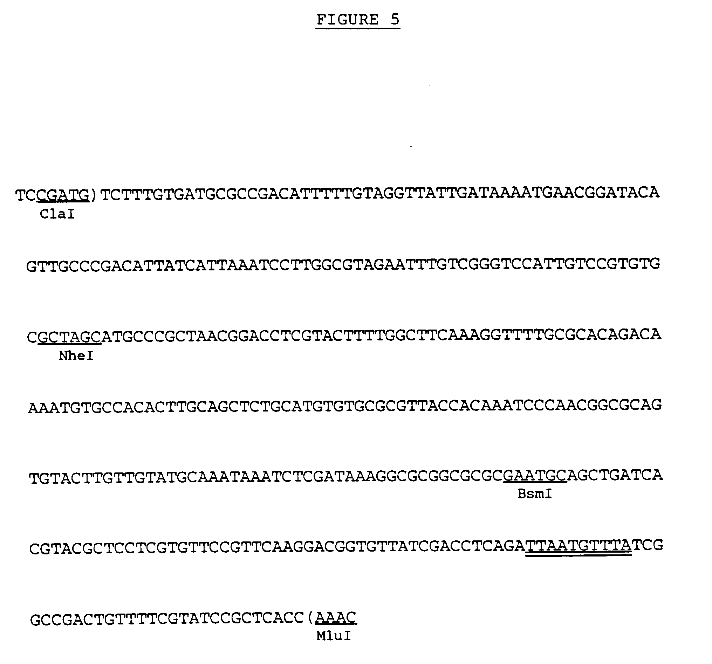

Les séquences SEQ.ID.NO:1 et SEQ.ID.NO:2 des fragments AMIG et STAB ont été analysées : les figures 4 et 5 représentent ces séquences, où sont indiquées les régions présentant des caractéristiques particulières :
- les séquences des sites de restriction bordant les fragments sont soulignées, et sont séparées par des parenthèses de la séquence du reste des fragments ; les séquences des sites de restriction à l'intérieur des fragments sont soulignées ;
- les séquences similaires aux séquences consensus CEN de *S. cerevisae* sont en caractères gras ;
- les séquences similaires aux séquences consensus ARS de levure sont soulignées d'un double trait ;

La séquence AMIG (Figure 4) possède un segment (MCCS) qui contient une homologie totale avec les 11 paires de bases des éléments ARS, et 4 segments présentant une homologie de 10 paires de bases sur 11 avec la séquence consensus coeur des éléments ARS et un segment qui présente une homologie de 9 paires de base sur 11 avec la séquence consensus ARS(C) située en amont du domaine A dans les éléments ARS de levure. Toutes ces séquences sont comprises dans un segment de 419 paires de bases qui contient environ 70% de A+T. D'autre part, la séquence AMIG présente un très grand nombre de motifs ATTA ou ATTTA, et présente également 2 séquences très similaires à la séquence consensus du site de fixation du facteur de transcription NFI (facteur nucléaire I). L'analyse de la séquence AMIG révèle également des régions très similaires aux séquences consensus CEN de *S. cerevisae*. Une de ces séquences, qui est localisée de la position 41 à la position 47 (la numérotation des bases se réfère à celle indiquée dans la liste des séquences) présente 100% d'homologie avec la séquence consensus CDEI.

En outre, les séquences localisées des positions 177 à 263 et 158 à 166 sont également très semblables aux séquences consensus CDEII et CDEIII des éléments ARS de levure. Cependant, si l'on compare l'orientation du bloc CDEII/CDEIII par rapport à CDEI dans le plasmide pEI1néo et dans les éléments ARS de levure, l'on constate que ces orientations sont inversées.

La séquence STAB (Figure 5) présente également des motifs ATTA ou ATTTA, et des séquences très similaires aux séquences consensus des sites de fixation des facteurs de transcription NFI (facteur nucléaire I) et NFIII, ainsi qu'une séquence de type ARS.

### EXEMPLE 3 : CONSTRUCTION D'UN PLASMIDE PORTANT LA SEQUENCE AMIG ET EXPRIMANT LE GENE NéoR SOUS CONTROLE DU PROMOTEUR IEO

Le promoteur *IEO* du baculovirus *Ac*MNPV a été amplifié par PCR sous forme d'un fragment flanqué par des sites de restriction KpnI et BssHII. Son extrémité BsslIII a été réparée à la polymérase de Klenow, puis il a été inséré à la place du fragment KpnI-SmaI de pUC19 donnant le plasmide pIEO.

Le fragment BglII-BamHI de pnéo qui contient le gène NéoR a ensuite été inséré dans le site BamlII de pIEO pour permettre l'expression du gène *Néo*R sous contrôle du promoteur *IEO*, donnant le plasmide pIEONéoR.

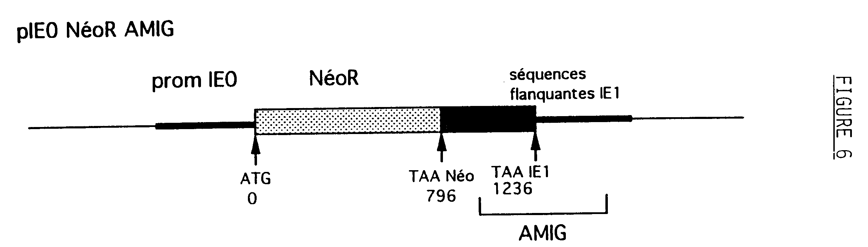

Le fragment HincII-BamHI de pIE1 qui contient la séquence AMIG a été inséré à la place du fragment SmaI-BamHI de pIEONéoR donnant le plasmide pIEONéoRAMIG. Cette construction est représentée à la figure 6.

Les cellules *Sf*9 ont été transformées par lipofection avec la construction pIEONéoR AMIG et les cellules résistantes sélectionnées par du G418 à 1mg/ml comme décrit à l'exemple 1.

Après 10 passages, l'ADN de ces cellules, extrait par lyse alcaline, a été analysé par transfert de Southern comme décrit à l'exemple 1. L'information génétique est là aussi présente sous la forme d'un épisome de haut poids moléculaire constitué de nombreuses copies en tandem du plasmide pIEONéoR AMIG.

### EXEMPLE 4 : EXPRESSION SOUS CONTROLE D'UN PROMOTEUR VIRAL D'UN GENE PORTE PAR UN VECTEUR COMPRENANT LES SEQUENCES AMIG ET STAB DANS LA DESCENDANCE DES CELLULES TRANSFORMEES.

Un fragment HindIII-XhoI contenant le promoteur IEN de AcNPV a été obtenu selon le protocole suivant
- Un lieur XhoI de 8 pb a été inséré au site BglII de pNéo en -34 de l'ATG du gène NéoR. Ceci a donné le plasmide pNéoXhoI
- Le plasmide pUC18IEN a été construit par insertion du fragment PstI-N de 2620 pb de AcNPV (qui contient le gène *IEN* et son promoteur) au site PstI de pUC18. Le fragment BglII-PstI de pUC18IEN promoteur *IEN* a été sous-cloné dans le plasmide pSelect (PROMEGA). Un site unique XhoI a été créé au niveau du codon d'initiation ATG de *IEN* par mutagénèse dirigée.

Le fragment HindIII-XhoI de pUC18IEN contenant le promoteur *IEN* a été inséré à la place du fragment HindIII-XhoI du plasmide pBLCAT2 (LUCKOW and SCHUTZ, Nucleic Acid Res., 15, (13) 5940 (1987)). Le plasmide ansi obtenu est dénommé pIENCAT.

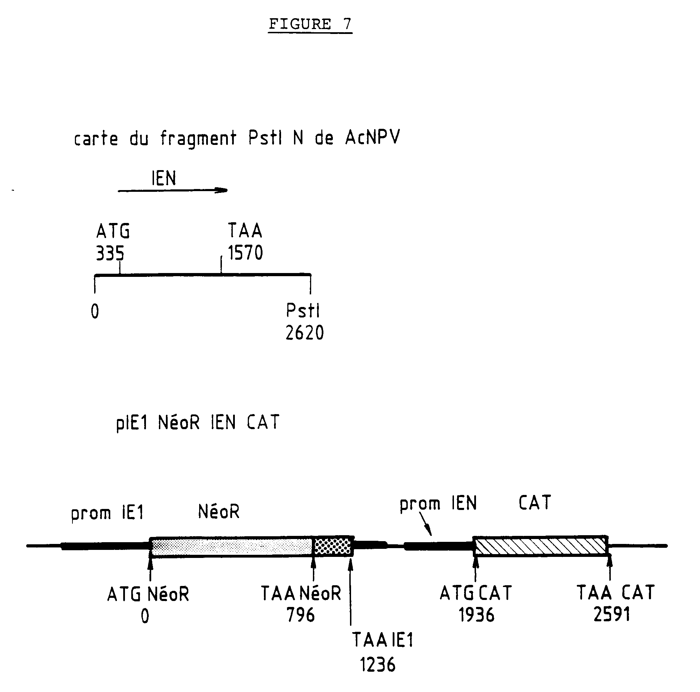

L'extrémité HindIII du fragment HindIII-SacI de pIENCAT comprenant le promoteur *IEN* et le gène *CAT* a été réparée par la polymérase de Klenow puis le fragment a été inséré entre les sites SmaI et SacI du plasmide pIElNéoR pour donner le plasmide pIE1NéoRIENCAT. Cette construction est représentée à la figure 7.

Les cellules Sf9 ont été transformées (comme décrit à l'Exemple 1 par lipofection avec la construction pIE1NéoRIENCAT, et les cellules résistantes sélectionnées par du G418 à 1 mg/ml.

Après 10 passages, les cellules ont été séparées en 2 lots : l'un a été maintenu sous pression de sélection et l'autre non. Après 15 passages supplémentaires, ces deux lots de cellules ont été analysés par électrophorèse en champ pulsé.

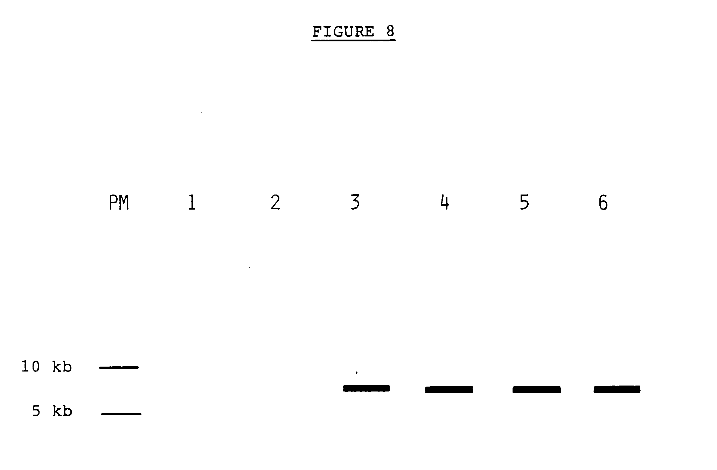

Les résultats sont représentés à la Figure 8 ; l'information génétique est toujours présente sous la forme d'un épisome de haut poids moléculaire constitué de nombreuses copies en tandem du plasmide pIE1NéoRIENCAT, et ceci que les cellules soient maintenues ou non sous pression de sélection. De plus, le nombre de copies du plasmide est comparable dans les deux lots de cellules Légende de la Figure 8 :
PM : marqueur de poids moléculaire (en pb)
Piste 1 : Cellules Sf9 non transformées, digestion BglII
Piste 2 : Cellules Sf9 non transformées, digestion HindIII
Piste 3 : Cellules Sf9 transformées avec pIElNéoRIENCAT, pression de sélection, digestion BglII (un seul site dans pIE1NéoRIENCAT) ;
Piste 4 : Cellules Sf9 transformées avec pIElNéoRIENCAT, pression de sélection, digestion HindIII (un seul site dans pIE1NéoRIENCAT) ;
Piste 5 : Cellules Sf9 transformées avec pIE1NéoRIENCAT, sans pression de sélection, digestion BglII ;
Piste 6 : Cellules Sf9 transformées avec pIE1NéoRIENCAT, sans pression de sélection, digestion HindIII.

En outre, le dosage par ELISA, sur un extrait cellulaire total en utilisant un anticorps anti-CAT (BOEHRINGER), de la CAT produite par les cellules donne des résultats identiques pour les deux lots de cellules comme le montre le tableau I ci-dessous.

Ces résultats montrent que la structure épisomale est stable, y compris en l'absence de pression de sélection.

**TABLEAU I**

| | Concentration de CAT (ng/µg de protéines cellulaires) |
|---|---|
| 25 passages (+G418) | 0,25 |
| 10 passages (+G418) + 15 passages (-G418) | 0,26 |

### EXEMPLE 5 : EXPRESSION SOUS CONTROLE D'UN PROMOTEUR CELLULAIRE D'UN GENE PORTE PAR UN VECTEUR PLASMIDIQUE COMPRENANT LES SEQUENCES AMIG ET STAB DANS LA DESCENDANCE DES CELLULES TRANSFORMEES.

L'extrémité XhoI du fragment XhoI-SacI du plasmide pCaSpcR-hs [THUMMEL et PIROTTA, Dros. inf. Sem. 71 : 150 (1992)] a été réparée par la polymérase de Klenow. Ce fragment contient le promoteur *hsp70* de *Drosophila melanogaster* et environ 1000 pb des séquences 3' de ce gène. Ces deux éléments sont séparés par un multi-site de clonage comportant en particulier les sites BglII et StuI. Ce fragment a été inséré à la place du fragment SacI-SmaI de pIElNéoR pour donner le plasmide pIE1NéoR hsp.

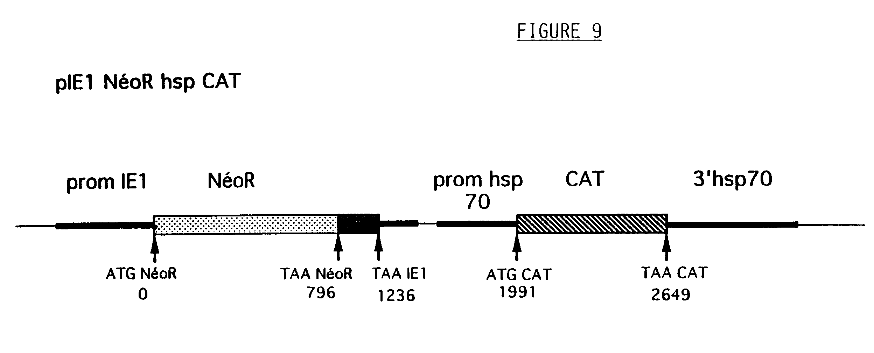

Le fragment BglII HincII du plasmide pBLCAT2 qui contient le gène CAT a été inséré entre les sites BglII et StuI de pIE1NéoRhsp pour donner le plasmide pIE1NéoRhspCAT. Cette construction est représentée à la figure 9.

Les cellules *Sf9* ont été transformées par lipofection avec la construction pIE1NéoRhspCAT et les cellules résistantes sélectionnées par du G418 à 1mg/ml comme décrit à l'exemple 1.

Après 10 passages, les cellules ont été séparées en 2 lots : l'un maintenu en pression de sélection et l'autre non. Après 5 passages supplémentaires, l'ADN de ces deux lots de cellules a été extrait par lyse alcaline et analysé par transfert de Southern comme décrit à l'exemple 1. L'information génétique est là aussi présente sous la forme d'un épisome de haut poids moléculaire constitué de nombreuses copies en tandem du plasmide pIE1NéoRhspCAT.

Dans les cellules de Drosophile, le promoteur *hsp70* est inductible sous l'effet d'un choc thermique. Les cellules *Sf9* transformées avec pIE1NéoRhspCAT ont donc été soumises à un choc thermique de 0,5, 1 ou 2 heures à 37°C et la quantité de CAT produite a été dosée par ELISA comme décrit à l'exemple 4.

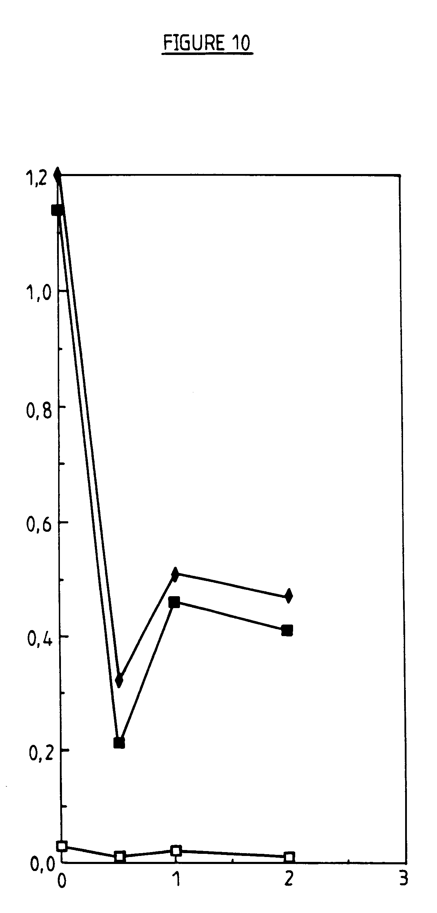

Les résultats sont présentés figure 10.

Légende de la figure 10 :
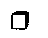 Cellules Sf9 non transformées
♢ Cellules Sf9 transformées avec pIE1NéoRhspCAT ;
10 passages sans pression de sélection
■ Cellules Sf9 transformées avec pIE1NéoRhspCAT ;
10 passages sous pression de sélection.

La durée du choc thermique à 37°C (en heures) est représentée en abscisse ; la quantité de CAT produite (en ng/mg de protéines totales) est représentée en ordonnée.

Ces résultats montrent que :
- Les cellules se comportent exactement de la même façon qu'elles soient maintenues sous pression de sélection ou non.
- La quantité de CAT produite sous contrôle du promoteur hsp 10 (1,2 ng/µg de protéines totales) est beaucoup plus importante que sous contrôle du promoteur IEN (0,25 ng/mg de protéines totales : cf. Tableau I, exemple 4). Cependant, la production n'est pas inductible par choc thermique ; au contraire, elle diminue d'un facteur 2 à 3.

### EXEMPLE 6 : EXPRESSION D'UN GENE PORTE PAR UN VECTEUR COMPRENANT LES SEQUENCES AMIG ET STAB, SOUS CONTROLE D'UN PROMOTEUR VIRAL TRANS-ACTIVE ET CIS-ACTIVE PAR DES ELEMENTS VIRAUX.

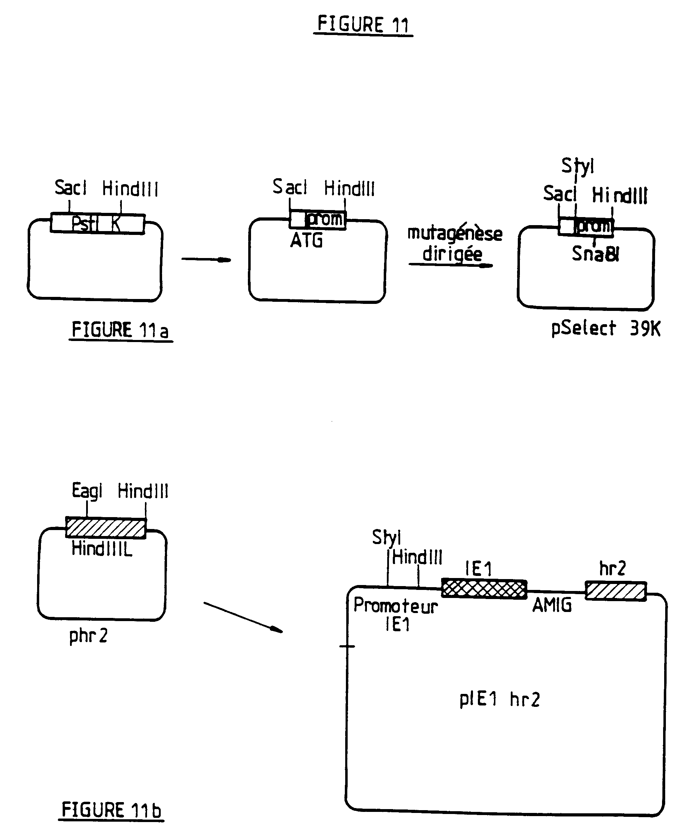

Le promoteur précoce *39K* du Baculovirus *Ac*MNPV est *trans*-activé par la protéine IE1, produit du gène *IE1* [GUARINO et SUMMERS, Virol., 57 : 563-571 (1986)] et cis activé par la séquence hr2 [GUARINO et SUMMERS, Virol. 60 : 215-223 (1986) ; et Virol. 60 : 224-229, (1986)]. Les Inventeurs ont réalisé l'expression stable du gène CAT sous contrôle de ce promoteur en faisant la construction suivante :
- Le fragment PstI-K de *Ac*MNPV contenant le gène *39K* a été inséré dans le site PstI de pUC18. Le fragment SacI-HindIII du plasmide ainsi obtenu contient le promoteur *39K* et le début de la séquence codante du gène. Il a été inséré à la place du fragment SacI-HindIII de pSelect (PROMEGA). Un site unique StyI a été créé au niveau de l'ATG de *39K* par mutagénèse dirigée, pour donner le plasmide pSelect39K. Les étapes de cette construction sont représentées à la figure 11a.
- D'autre part, le fragment HindIII-L de AcMNPV contenant hr2 a été inséré au site HindIII de pUC18 donnant le plasmide phr2.
- Le fragment EagI-HindIII de phr2 contient la totalité des séquences hr2. Ses extrémités ont été réparées par la polymérase de Klenow et ce fragment a été inséré dans le site XbaI de pIE1 dont les extrémités ont également été réparées par la polymérase de Klenow. Le plasmide obtenu est dénommé pIE1hr2. Les étapes de cette construction sont représentées à la figure 11b.
- Le plasmide pIElhr2 a été digéré par HindIII et StyI pour éliminer les sites correspondant à ces enzymes et refermé après réparation des extrémités à la polymérase de Klenow.
- Le fragment SnaBI-SacI de pSelect39K contenant le promoteur *39K* muté, et un site StyI a été inséré à la place du fragment SnBI-SacI du plasmide pIElhr2, donnant le plasmide pIE1hr239K-1.
- Un lieur BglII a été inséré au site StyI de pIE1hr239K-1.
- Le fragment SacI-HpaI de pBLCAT2 [LUCKOW et SCHULLZ, Nucl. Acids Res. 15 : 5490 (1987)] contient les séquences de polyadénylation du gène de l'antigène T de SV40. Il a été inséré à la place du fragment BspEI-SacI de pIE1hr239K-1 après réparation de l'extrémité BspEI à la polymérase de Klenow. Le plasmide obtenu est dénommé pIE1hr239K-2.

Pour éliminer le site BamHI de pIE1hr239K-2, ce plasmide a été digéré par BamHI et refermé après réparation des extrémités à la polymérase de Klenow. Puis un lieur BamHI de 10 pb a été inséré au site SacI de ce plasmide, pour donner le plasmide pIE1hr239K-3.

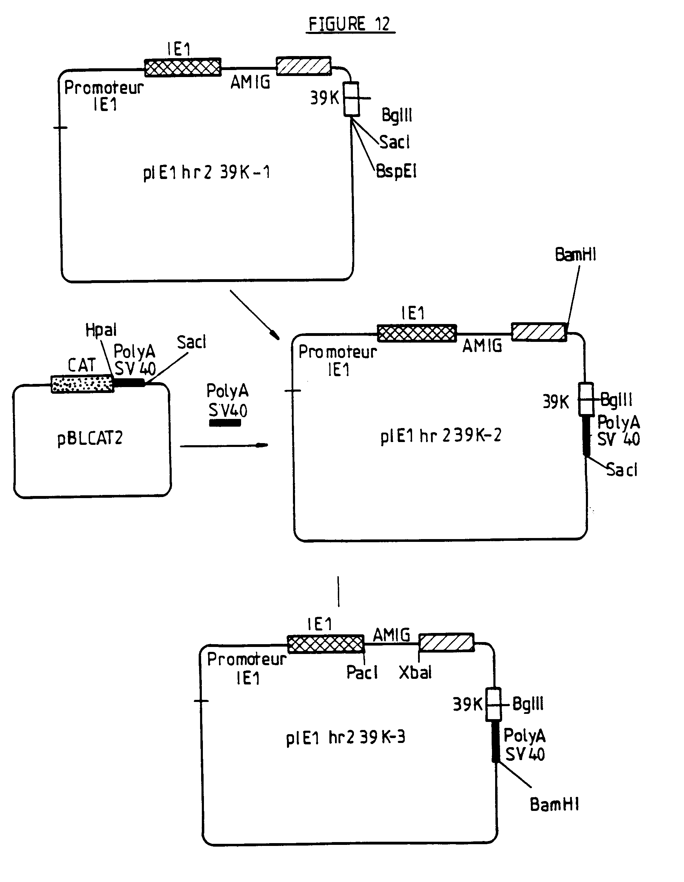

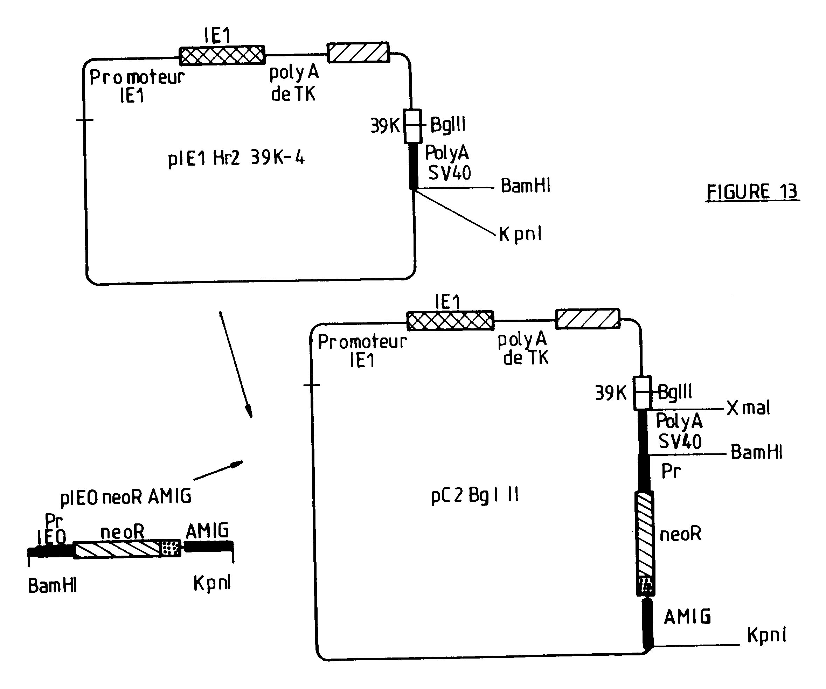

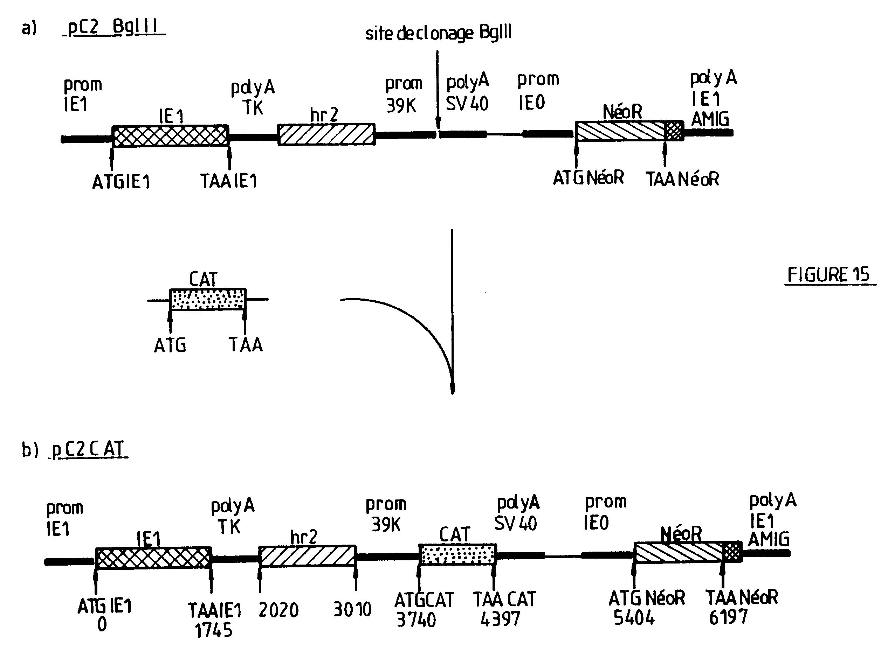

Les étapes de la construction de pIE1hr239K-1, pIElhr239K-2, pIElhr239K-3 sont schématisées à la figure 12.
- Le fragment PacI-XbaI de pIE1hr239K-3 contient les séquences de polyadénylation de *IE1* et la séquence AMIG. Après réparation des extrémités Bsu36I et PacI à la polymérase de Klenow, il a été remplacé par le fragment Bsu36I-AvrII de pBKCMV (STRATAGENE) qui contient les séquences de polyadénylation du gène de la thymidine kinase du virus CMV. Le plasmide obtenu est dénommé pIE1hr239K-4.
- Le fragment BamHI-KpnI de pIEONéoRAMIG a été inséré à la place du fragment BamHI-KpnI de pIE1hr239K-4, donnant le vecteur d'expression pC2BglII dans lequel on peut insérer un gène étranger sous contrôle du promoteur *39K* dans le site unique BglII. Les étapes de la construction du vecteur pc2 BglII sont représentées à la figure 13. Une carte plus détaillée de pC2BglII est représentée à la figure 15a.
- Le fragment XmaI-BgllII de pBLCAT2 contenant le gène CAT a été inséré à la place du fragment XmaI-BglII de pC2BglII donnant le plasmide pC2CAT.

pC2CAT permet l'expression des gènes :
- *NéoR* sous contrôle du promoteur *IEO* (séquence polyA : *IE1*)
- *IE1* sous contrôle du promoteur *IE1* (séquence polyA : *TK* CMV)
- *CAT* sous contrôle du promoteur *39K trans*-activé par IE1 et *cis*-activé par hr2 (séquence polyA : AgT SV40).

La séquence STAB est présente en amont de *IE1* et la séquence AMIG en aval de *NéoR*. Ainsi, elles encadrent les séquences codantes du vecteur d'expression.

Des cellules *Sf9* ont été transformées par lipofection avec la construction pC2CAT et les cellules résistantes sélectionnées par du G418 à 1 mg/ml comme décrit à l'exemple 1.

Après 10 passages, l'ADN de ces cellules a été extrait par lyse alcaline et analysé par transfert de Southern. L'information génétique est là aussi présente sous la forme d'un épisome de haut poids moléculaire constitué de nombreuses copies en tandem du plasmide pC2CAT. La production de CAT mesurée par dosage ELISA est de 1,6 ng/mg de protéines totales.

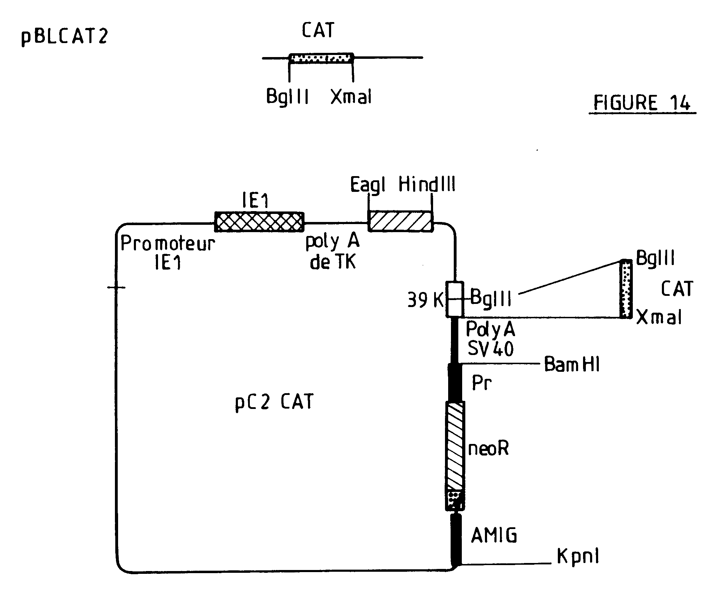

Le vecteur pC2CAT est représenté à la figure 14, et de façon plus détaillée, à la figure 15 b.

LISTE DE SEQUENCES

INFORMATION GENERALE :

(i) DEPOSANTS:
(A) NOM: INSTITUT NATIONAL DE LA RECHERCHE
AGRONOMIQUE - INRA
(B) RUE: 147, rue de l'Université
(C) VILLE: PARIS
(D) PAYS: FRANCE
(E) CODE POSTAL: 75341 PARIS CEDEX 07

(A) NOM: CENTRE NATIONAL DE LA RECHERCHE
SCIENTIFIQUE - CNRS
(B) RUE: 3, rue Michel Ange
(C) VILLE: PARIS
(D) PAYS:FRANCE
(E) CODE POSTAL: 75794 PARIS CEDEX 16

(A) NOM: DEVAUCHELLE Gérard
(B) RUE: 137, Chemin de l'Espervette
(C) VILLE: SAINT CHRISTOL-LES-ALES
(D) PAYS: FRANCE
(E) CODE POSTAL: 30380

(A) NOM: CERUTTI Martine
(B) RUE:2997, Route de Montèze
(C) VILLE: SAINT CHRISTOL-LES-ALES
(D) PAYS: FRANCE
(E) CODE POSTAL: 30380

(A) NOM: PERSILLON Cécile
(B) RUE: 11, Rue de Bresis
(C) VILLE: ALES
(D) PAYS: FRANCE
(E) CODE POSTAL: 30100

(ii) TITRE DE L'INVENTION: SEQUENCES NUCLEOTIDIQUES
PERMETTANT LA REPLICATION ET LE MAITIEN D'UNE
INFORMATION GENETIQUE DANS LES CELLULES ANIMALES

(iii) NOMBRE DE SEQUENCES: 2

(iv) FORME LISIBLE PAR ORDINATEUR:
(A) TYPE DE SUPPORT: Floppy disk
(B) ORDINATEUR: IBM PC compatible
(C) SYSTEME D'EXPLOITATION: PC-DOS/MS-DOS
(D) LOGICIEL: PatentIn Release #1.0, Version #1.25 (OEB)

(iv) DONNEES DE LA DEMANDE ANTERIEURE:
(A) NUMERO DE DEPOT: FR93 09156
(B) DATE DE DEPOT: 26 JUILLET 1993

LISTE DE SEQUENCES

NOMBRE DE SEQUENCES: 2

(1) INFORMATION POUR LA SEQ ID NO: 1:

(i) CARACTERISTIQUES DE LA SEQUENCE:
(A) LONGUEUR: 516 paires de bases
(B) TYPE: acide nucléique
(C) NOMBRE DE BRINS: double
(D) CONFIGURATION: linéaire

(ii) TYPE DE MOLECULE: ADN (génomique)

(iii) HYPOTHETIQUE: NON

(iii) ANTI-SENS: NON

(vi) ORIGINE:
(A) ORGANISME: Autographa californica nuclear polyhedrosis
virus

(xi) DESCRIPTION DE LA SEQUENCE: SEQ ID NO: 1:

ACGTTAGTCA AGTAATTCAA AAATATAATA GATTTAAGCA TCACATGTTT GTAATCGGTA 60

AAGTGAACCG AAGAGAGAGC ACTACATTGC ACAATAATTT GTTAAAATTG TTAGCTTTAA 120

TATTACAGGG TCTGGTTCCG TTGTCCGACG CTATAACGTT TGCGGAACAA AAACTAAATT 180

GTAAATATAA AAAATTCGAA CTTTAATTAA TTATACATAT ATTTTGAATT TAATTAATTA 240

TACATATATT TTATATTATT TTTGTCTTTT ATTATCGAGG GGCCGTTGTT GGTGTGGGGT 300

TTTGCATAGA AATAACAATG GGAGTTGGCG ACGTTGCTGC GCCAACACCA CCTCCTTCCT 360

CCTCCTTTCA TCATGTATCT GTAGATAAAA TAAAATATTA AACCTAAAAA CAAGACCGCG 420

CCTATCAACA AAATGATAGG CATTAACTTG CGTGACGCTG TCACTAACGT TGGACGATTT 480

GCCGACTAAA CCTTCATCGC CCAGTAACCA ATCTAG 516

(2) INFORMATION POUR LA SEQ ID NO: 2:

(i) CARACTERISTIQUES DE LA SEQUENCE:
(A) LONGUEUR: 387 paires de bases
(B) TYPE: acide nucléique
(C) NOMBRE DE BRINS: double
(D) CONFIGURATION: linéaire

(ii) TYPE DE MOLECULE: ADN (génomique)

(iii) HYPOTHETIQUE: NON

(iii) ANTI-SENS: NON

(vi) ORIGINE:
(A) ORGANISME: Autographa californica nuclear polyhedrosis
virus

(xi) DESCRIPTION DE LA SEQUENCE: SEQ ID NO: 2:

GATGTCTTTG TGATGCGCCG ACATTTTTGT AGGTTATTGA TAAAATGAAC GGATACAGTT 60

GCCCGACATT ATCATTAAAT CCTTGGCGTA GAATTTGTCG GGTCCATTGT CCGTGTGCGC 120

TAGCATGCCC GCTAACGGAC CTCGTACTTT TGGCTTCAAA GGTTTTGCGC ACAGACAAAA 180

TGTGCCACAC TTGCAGCTCT GCATGTGTGC GCGTTACCAC AAATCCCAAC GGCGCAGTGT 240

ACTTGTTGTA TGCAAATAAA TCTCGATAAA GGCGCGGCGC GCGAATGCAG CTGATCACGT 300

ACGCTCCTCG TGTTCCGTTC AAGGACGGTG TTATCGACCT CAGATTAATG TTTATCGGCC 360

GACTGTTTTC GTATCCGCTC ACCAAAC 387

## Revendications

**1)** Utilisation d'un fragment d'ADN comprenant la séquence indiquée dans la liste des séquences en annexe sous le numéro SEQ.ID.N°1, pour l'obtention d'un vecteur capable de se répliquer de manière autonome dans une cellule animale et de se maintenir dans la descendance de ladite cellule sous forme d'épisome.

**2)** Utilisation selon la Revendication 1, caractérisée en ce qu'on utilise le fragment d'ADN de séquence SEQ.ID.N°1 en association avec un fragment d'ADN comprenant la séquence indiquée dans la liste des séquences en annexe sous le numéro SEQ.ID.N°2.

**3)** Vecteur capable de se multiplier dans une cellule animale et de se maintenir dans la descendance de ladite cellule, caractérisé en ce qu'il est sous forme d'épisome constitué par un concatémère d'un vecteur-unité, lequel vecteur-unité comprend la séquence indiquée dans la liste des séquences en annexe sous le numéro SEQ.ID.N°1.

**4)** Vecteur selon la Revendication 3, caractérisée en ce que ledit vecteur-unité comprend en outre la séquence indiquée dans la liste des séquences en annexe sous le numéro SEQ.ID.N°2.

**5)** Procédé d'obtention de lignées cellulaires animales capables de transmettre à leur descendance une information génétique extra-chromosomique d'origine hétérologue, lequel procédé est caractérisé en ce qu'il comprend une étape au cours de laquelle l'on procède à la transfection d'une cellule animale par une préparation d'ADN comprenant au moins un vecteur-unité portant ladite information génétique et comprenant la séquence indiquée dans la liste des séquences en annexe sous le numéro SEQ.ID.N°1, ou par une préparation d'ADN comprenant au moins un épisome constitué d'un concatémère dudit vecteur-unité, et une étape au cours de laquelle on procède à la multiplication de ladite cellule animale, et à la sélection des descendants de ladite cellule portant au moins un épisome constitué d'un concatémère dudit vecteur-unité.

**6)** Procédé selon la Revendication 5, caractérisé en ce que le vecteur-unité comprend en outre la séquence indiquée dans la liste des séquences en annexe sous le numéro SEQ.ID.N°2.

**7)** Lignée cellulaire stable capable d'exprimer une information génétique d'origine hétérologue, laquelle lignée est caractérisée en ce que chaque cellule de ladite lignée contient au moins un vecteur épisomal selon l'une quelconque des Revendications 3 ou 4.

**8)** Lignée cellulaire selon la Revendication 7, caractérisée en ce qu'il s'agit d'une lignée de cellules d'insectes, en particulier de lépidoptères.
